# FASCICULE DE BREVET EUROPEEN

(11) **EP 3 191 142 B1**
(45) Date de publication et mention de la délivrance du brevet: **17.07.2019**
(21) Numéro de dépôt: 15771187.0
(22) Date de dépôt: 07.09.2015
(51) Int. Cl.: A61L 15/60, A61L 15/12, A61L 15/42, C08L 1/08, C08L 1/26, C08L 33/20, C08L 1/24, C08L 5/04, C08L 1/28

(54) **FIBRE ELECTRONEGATIVE POUR SON UTILISATION DANS LA CICATRISATION DES PLAIES**
ELEKTRONEGATIVE FASER ZUR VERWENDUNG BEI DER HEILUNG VON WUNDEN
ELECTRONEGATIVE FIBRE FOR USE IN THE HEALING OF WOUNDS

(30) Priorité: 08.09.2014 FR 1458398
(43) Date de publication de la demande: 19.07.2017
(73) Titulaire: URGO RECHERCHE INNOVATION ET DEVELOPPEMENT, 21300 Chenôve (FR)
(72) Inventeur: PERNOT, Jean-Marc, 21000 Dijon (FR)
(74) Mandataire: Cabinet Plasseraud
(86) Numéro de dépôt international: PCT/FR2015/052366
(87) Numéro de publication internationale: WO 2016/038287

(56) Documents cités:
- WO-A1-96/10106
- WO-A1-2012/131263

## Description

La présente invention a pour objet un matériau comprenant au moins une fibre électronégative pour son utilisation dans la cicatrisation des plaies, et préférentiellement dans la détersion des plaies, notamment des plaies chroniques. L'invention a également pour objet un dispositif médical, tel qu'un pansement cicatrisant ou un bandage, comprenant ledit matériau.

Un pansement est un dispositif de protection permettant de recouvrir une plaie située sur la peau. Un pansement peut avoir plusieurs fonctions cumulables ou non, telles que:
- protéger la plaie (contre une infection, une irritation) et l'isoler du milieu extérieur ;
- permettre une meilleure cicatrisation en maintenant un milieu humide favorable sur le lit de la plaie ;
- faire cesser un saignement minime en comprimant les petits vaisseaux ;
- rapprocher les berges d'une plaie ;
- absorber les exsudats afin de préserver les berges de la plaie et la peau périlésionnelle.

Certains types de pansement sont donc spécifiquement développés pour favoriser la cicatrisation d'une plaie, tandis que d'autres n'ont pas cette vocation.

La cicatrisation naturelle d'une plaie se déroule en trois phases successives, chacune de ces phases étant caractérisée par des activités cellulaires spécifiques et différentes: la phase de détersion, la phase de bourgeonnement (ou granulation) et la phase d'épithélialisation. Tout au long du processus de cicatrisation, la plaie produit des exsudats fluides ou visqueux qui doivent si possible être absorbés par le pansement cicatrisant ou évacués par ce dernier pour être guidés vers un réservoir à l'extérieur de la plaie (cas de la thérapie par pression négative - TPN).

Les capacités de détersion naturelle de la plaie peuvent être insuffisantes lorsque le traumatisme est important ou lorsque le patient souffre de pathologies parallèles, comme les pathologies veineuses ou le diabète. Dans ces cas, on observe un allongement considérable de la durée de la phase de détersion conduisant à des plaies chroniques difficiles à soigner, comme par exemple l'ulcère de jambe.

Dans le cas de plaies pour lesquelles le processus de détersion naturelle est insuffisant, il est nécessaire d'éliminer le tissu fibrineux sans perturber la phase de bourgeonnement. L'élimination de ce tissu fibrineux est couramment désignée sous le terme de "détersion assistée" par opposition à la détersion naturelle.

Selon la technique utilisée, la détersion assistée peut être qualifiée de détersion mécanique ou chirurgicale, de détersion enzymatique, de détersion autolytique ou de détersion biologique.

La détersion mécanique ou chirurgicale est une technique rapide qui consiste à découper le tissu fibrineux, soit à l'aide d'un bistouri, de pinces, de ciseaux ou d'une curette de Brock, soit à l'aide d'appareils sophistiqués par jets d'eau sous pression ou excision au laser. Cette technique est réalisée au lit du patient ou en milieu chirurgical selon la gravité de la plaie. Cependant, cette technique est souvent douloureuse et peut entraîner des saignements et parfois même une hémorragie. Elle est alors traumatisante pour le patient. Elle nécessite aussi couramment une médication antalgique au préalable qui allonge la durée des soins.

La détersion autolytique consiste, quant à elle, à déposer sur la plaie des matériaux absorbants, tels que des pansements ou des bandages, à base de fibres gélifiantes particulières. Le but d'une telle technique est soit de parvenir à ramollir la fibrine pour permettre par la suite son retrait à l'aide d'une curette par du personnel médical ou paramédical compétent, soit de permettre son retrait par une action d'accroche de la fibrine au matériau absorbant, ou éventuellement d'associer ces deux méthodes de façon optimale.

Les matériaux classiquement utilisés pour faciliter la détersion sont des non tissés aiguilletés de fibres gélifiantes d'alginate ou de carboxyméthylcellulose.

Les fibres de carboxyméthylcellulose gélifient au contact des exsudats, permettant un ramollissement efficace de la fibrine, mais altérant considérablement la cohésion du matériau non tissé les comprenant, empêchant ainsi toute accroche lors du retrait du matériau.

Les fibres d'alginate permettent également de ramollir la fibrine, favorisant ainsi son retrait manuel. Toutefois, bien que les fibres d'alginate ne gélifient pas au contact des exsudats de façon aussi importante que les fibres de carboxyméthylcellulose, préservant ainsi la cohésion du matériau non tissé les comprenant, elles ne présentent aucune propriété d'accroche de la fibrine lors du retrait du matériau.

Un matériau permettant à la fois un ramollissement de la fibrine et une accroche de cette dernière lors de son retrait, tout en conservant sa cohésion, serait un compromis optimal.

Le document WO 2012/131263 propose précisément d'utiliser des fibres superabsorbantes, telles que celles commercialisées par la société TOYOBO qui présentent une capacité d'absorption des liquides de 27,8 g d'eau par gramme de fibre et une densité de charges négatives de 12,3 mmol/g, pour l'obtention d'un non tissé permettant de ramollir la fibrine et de favoriser son accroche lors de son retrait. Cependant, les propriétés superabsorbantes des fibres utilisées imposent un aiguilletage suffisamment espacé pour permettre le passage des exsudats malgré le gonflement des fibres. L'aiguilletage trop lâche pose toutefois des problèmes de cohésion du non tissé, dont les fibres se détachent et viennent polluer la plaie. Pour éviter ce phénomène, la demande WO 2012/131263 propose d'utiliser d'un non tissé comprenant un mélange de fibres thermoliantes non absorbantes et de fibres superabsorbantes, auxquelles est associée une couche contact spécifique. Un tel produit demeure compliqué et coûteux à fabriquer. Le document WO 96/10106 décrit une composition comprenant une fibre formée par co-spinning comprenant un alginate et un polymère cellulosique tel que la carboxyméthylcellulose.

Il serait donc souhaitable de disposer d'un matériau simple et facile à fabriquer, pour le soin des plaies, notamment des plaies chroniques, qui présente une bonne capacité de détersion autolytique, c'est-à-dire qui permette une action d'accroché de la fibrine optimisée pour diminuer ou éliminer le recours à des actes chirurgicaux, sans toutefois altérer l'absorption et la diffusion des exsudats.

De manière surprenante, la Demanderesse a découvert qu'il était possible de répondre à ces problématiques au moyen d'un matériau à base d'une fibre électronégative présentant une capacité d'absorption des fluides et une densité de charges négatives spécifiques.

Ainsi, l'invention a pour objet, selon un premier aspect, un matériau comprenant au moins une fibre électronégative présentant une densité de charges négatives comprise entre 0,01 et 5 mmol/g et une capacité d'absorption des liquides comprise entre 10 et 20 g de sérum physiologique par gramme de fibre pour son utilisation dans la cicatrisation des plaies, préférentiellement dans la détersion des plaies, et notamment des plaies chroniques.

L'invention a également pour objet, selon un deuxième aspect, un dispositif médical, tel qu'un pansement cicatrisant ou un bandage, comprenant un tel matériau.

La Demanderesse a en effet constaté que le matériau selon l'invention ou le dispositif médical le mettant en oeuvre garantissent le retrait de la fibrine nécessaire pour une bonne détersion autolytique. En effet, les inventeurs ont notamment mis en évidence qu'en utilisant des fibres électronégatives présentant une capacité d'absorption des fluides particulière, il était possible d'obtenir un matériau accrochant la fibrine, et permettant son retrait lors du retrait du matériau, tout en favorisant l'absorption et la diffusion des exsudats.

La présente invention permet ainsi d'optimiser la cicatrisation des plaies, notamment des plaies chroniques, en proposant pour la première fois un matériau qui favorise simultanément la détersion autolytique et l'absorption et la diffusion des exsudats liquides sans générer de phénomène de « gel blocking ».

Enfin le matériau de l'invention présente, en outre, l'avantage d'être cohésif et de ne pas se déchirer quand on le retire.

### Fibre électronégative

Le matériau selon la présente invention comprend au moins une fibre électronégative présentant une densité de charges négatives comprise entre 0,01 et 5 mmol/g, de préférence entre 0,05 et 4 mmol/g, et plus préférentiellement entre 0,1 et 2 mmol/g.

On entend par « fibre électronégative » une fibre porteuse de charges négatives ou de groupements donneurs d'électrons, par exemple présentant des doublets d'électrons libres.

On entend par « densité de charges négatives » au sens de la présente demande, la quantité molaire des charges négatives portées par un gramme fibre, exprimée en mmol/g.

Les fibres utilisées dans le matériau de l'invention présentent par ailleurs une capacité d'absorption des liquides comprise entre 10 et 20 g de sérum physiologique par gramme de fibre, de préférence entre 11 et 15 g de sérum physiologique par gramme de fibre.

Le sérum physiologique au sens de la présente demande est une solution à 0,9% de chlorure de sodium NaCl.

Les fibres selon l'invention sont dites faiblement absorbantes ou absorbantes. Elles se distinguent donc en particulier des fibres qualifiées de « superabsorbantes », présentant une capacité d'absorption des liquides supérieure à 20 g d'eau (ou de solution saline telle que du sérum physiologique) par gramme de fibre, telles que les fibres TOYOBO mises en oeuvre dans la demande WO 2012/131263.

Les mesures d'absorption de sérum physiologique par une fibre ou un textile comprenant ces fibres peuvent par exemple être réalisées avec du sérum physiologique comprenant 0,9% de NaCl tel que décrit précédemment en appliquant les procédures décrites dans les méthodes EDANA 440.1.99.

La fibre électronégative selon l'invention est de préférence une fibre polymérique. Le polymère constituant la fibre peut notamment être choisi parmi les polyesters, les polyamides, les polyoléfines et leurs copolymères, les polymères acryliques, le polyuréthane, les polyacrylates et leurs copolymères, les polymères cellulosiques, et des mélanges de ceux-ci.

Selon un mode préféré de réalisation, le polymère constituant la fibre électronégative selon la présente demande est un polymère cellulosique, de préférence un dérivé cellulosique ou de la viscose ou un polymère acrylique, de préférence un copolymère d'acrylonitrile et de chlorure de vinyle.

Une fibre présentant la densité de charges négatives souhaitée selon la présente demande peut être obtenue par incorporation de particules ayant des propriétés échangeuses de cations ou de polyélectrolytes lors de la fabrication de ladite fibre.

L'introduction des particules ayant des propriétés échangeuses de cations ou des polyélectrolytes dans la matrice polymérique peut être réalisée lors de la synthèse du polymère, ou lors du filage du polymère par incorporation desdites particules ou desdits polyélectrolytes dans la formulation d'alimentation du dispositif de filage.

Selon un mode préféré de réalisation, les fibres électronégatives utilisées dans le matériau de l'invention sont obtenues par incorporation de résines échangeuses d'ions porteuses de groupements sulfonate et/ou par incorporation de polyélectrolytes porteurs de groupements carboxylate dans une matrice polymérique de viscose, telles que celles commercialisées par la société Kelheim sous les dénominations Poseidon ou Verdi. La demande de brevet US 2006/0246285 décrit un tel procédé de fabrication.

Alternativement, la fibre présentant la densité de charges négatives souhaitée selon la présente demande peut être obtenue par synthèse chimique d'un polymère présentant des groupements ioniques appropriés.

Selon un mode préféré de réalisation, les fibres électronégatives utilisées dans le matériau de l'invention sont obtenues par réaction d'un polymère modacrylique, et en particulier d'un copolymère acrylonitryle/chlorure de vinyle, avec une amine présentant un groupement échangeur d'ions. De telles fibres sont notamment commercialisées par la société Kaneka sous les références commerciales Kanecaron ion exchange fiber®. La demande de brevet EP 2 703 556 décrit un tel procédé de fabrication.

La densité de charges négatives des fibres selon l'invention est bien connue de l'homme du métier, par exemple des documents Chemical Fibers International 1/2014 « Ionically acitvated viscose fibers-properties and applications", Roland Scholz ou 52 nd Man-Made Fibers Congress Dornbirn "Ionically activated viscose fibers and their applications". La densité de charges négatives est, par ailleurs, usuellement indiquée sur les fiches techniques des fibres électronégatives disponibles dans le commerce.

### Matériau

Le matériau selon l'invention peut se présenter sous la forme d'un matériau tissé, non tissé, tricoté, d'un fil, d'une pelote, ou d'un amas de fibres.

De façon préférée, le matériau selon l'invention se présente sous la forme d'un matériau choisi de préférence parmi la liste constituée des non tissés, des tissés, ou des tricots.

L'assemblage des fibres peut de préférence être réalisé dans des conditions permettant d'obtenir un matériau textile de fort grammage, c'est-à-dire des matériaux dont le grammage est supérieur à 75 g/m2.

### Couche contact

Selon un mode particulier de réalisation, et sous réserve de ne pas altérer ses bonnes propriétés d'accroche de la fibrine et de cohésion, le matériau selon l'invention peut être partiellement recouvert d'une couche contact sur la face du matériau destinée à venir en contact avec la plaie, ladite couche comprenant des ouvertures permettant le passage des exsudats de la plaie.

Avantageusement, la couche contact est dite micro-adhérente, c'est-à-dire qu'elle permet de fixer provisoirement le matériau de l'invention sur la plaie. L'ensemble peut ensuite être retiré sans que la structure de la plaie ou de la peau périlésionnelle ne soit altérée, si bien que ledit ensemble est repositionnable et facilite les soins infirmiers. Cette fixation provisoire peut également aider le personnel soignant ou l'utilisateur à fixer le matériau à l'aide d'autres moyens de fixation, par exemple à recouvrir le matériau d'un moyen de contention ou d'un ruban adhésif. Dans ce cas, la couche contact peut être choisie de telle sorte qu'elle présente un pouvoir adhésif sur plaque d'acier compris entre 0,5 et 100 cN/cm, de préférence entre 5 et 40 cN/cm. Ce pouvoir adhésif est mesuré selon la méthode EN 1939 dans laquelle un échantillon de couche contact de 20 mm de large et 150 mm de long est posé sur une plaque d'acier et dans laquelle on mesure, au bout de 10 minutes, le pouvoir adhésif avec un dynamomètre à une vitesse de traction de 100 mm/min avec un angle de 90°.

La couche contact peut de préférence être formée d'une composition comprenant une matrice élastomérique et des hydrocolloïdes, et en particulier une matrice élastomérique dans laquelle des hydrocolloïdes sont de préférence dispersés de façon homogène.

La proportion d'hydrocolloïdes est de préférence comprise entre 2 et 20% en poids du poids de ladite composition.

La couche contact peut en particulier recouvrir entre 55 et 65% de la face la face de l'enveloppe destinée à venir en contact avec la plaie.

La couche contact présente de préférence un grammage allant de 110 à 500 g/m², de préférence de 150 à 200 g/m².

La couche contact permet avantageusement de ne pas adhérer à la plaie et d'éviter toute douleur au retrait du matériau de cicatrisation. En maintenant un milieu humide à la surface de la plaie tout en évitant le contact avec le matériau non tissé, elle améliore la cicatrisation. L'incorporation d'hydrocolloïdes confère à la composition élastomère un caractère hydrophile et favorise la vectorisation d'actifs susceptibles de favoriser le traitement de la plaie.

Ladite composition comprend un ou plusieurs élastomères choisis parmi les polymères séquencés poly(styrène-oléfine-styrène). Les copolymères séquencés utilisés dans le cadre de l'invention sont avantageusement des copolymères triblocs du type ABA comportant deux blocs terminaux thermoplastiques A styrène et une séquence centrale élastomère B qui est une oléfine, éventuellement associés à des copolymères diblocs du type AB comportant un bloc thermoplastique A styrène et une séquence élastomère B qui est une oléfine. Les séquences B oléfines de ces copolymères peuvent être constituées d'oléfines insaturées comme par exemple isoprène ou butadiène ou d'oléfines saturées comme par exemple éthylène-butylène ou éthylène-propylène.

Dans le cas d'un mélange de copolymères triblocs ABA et de copolymères diblocs AB, on pourra employer des mélanges de copolymères triblocs ABA et de copolymères diblocs AB commerciaux déjà disponibles ou réaliser des mélanges en toute proportion préalablement choisie à partir de deux produits disponibles indépendamment.

Les copolymères triblocs à séquence centrale insaturée sont bien connus de l'homme de l'art et sont notamment commercialisés par la société KRATON POLYMERS sous la dénomination KRATON® D.

Comme exemples de copolymères poly(styrène-isoprène-styrène) (en abrégé SIS) on peut ainsi citer les produits commercialisés sous les dénominations KRATON® D1107 ou KRATON® D1119 BT ou encore les produits commercialisés par la société EXXON MOBIL CHEMICAL sous la dénomination VECTOR® comme par exemple le produit commercialisé sous la dénomination VECTOR® 4113. Comme exemple de copolymères poly(styrène-butadiène-styrène) le produit commercialisé sous la dénomination KRATON® D1102.

Comme exemples de mélanges commerciaux de copolymères triblocs ABA et diblocs AB dans lesquels B est de l'isoprène, on peut citer les produits commercialisés par la société EXXON MOBIL CHEMICAL sous la dénomination VECTOR® 4114.

Tous ces copolymères à base d'isoprène ou de butadiène présentent généralement une teneur en styrène comprise entre 10 et 52% en poids rapportée au poids total dudit copolymère.

Dans le cadre de la présente invention, on préférera utiliser les copolymères séquencés triblocs poly(styrène-isoprène-styrène) (en abrégé SIS) ayant une teneur en styrène comprise entre 14 et 52 % et de préférence entre 14 et 30 % en poids rapporté au poids dudit poly(SIS).

D'une façon préférée, on utilisera pour réaliser les compositions de la présente invention des copolymères séquencés triblocs et en particulier le produit commercialisé par la société KRATON POLYMERS sous la dénomination KRATON® D1119 BT.

Les copolymères triblocs à séquence centrale saturée sont également bien connus de l'homme de l'art et sont par exemple commercialisés :
- par la société KRATON POLYMERS sous la dénomination KRATON® G, et en particulier sous la dénomination KRATON® G1651, KRATON® G1654 ou KRATON® G1652 pour les copolymères séquencés poly(styrène-éthylène-butylène-styrène) (en abrégé SEBS);
- par la société KURARAY sous la dénomination SEPTON® pour les copolymères séquencés poly(styrène-éthylène-propylène-styrène) (en abrégé SEPS).

Comme exemple de mélanges commerciaux de copolymères triblocs et diblocs, on peut citer le produit commercialisé par la société KRATON POLYMERS sous la dénomination KRATON® G1657 dont la séquence oléfine est éthylène-butylène.

Comme exemple d'un mélange particulier de copolymères triblocs et diblocs que l'on peut réaliser dans le cadre de la présente invention, on peut citer le mélange :
- d'un SEBS triblocs, comme en particulier le produit commercialisé par la société KRATON POLYMERS sous la dénomination KRATON® G1651 ; et
- d'un copolymère diblocs poly(styrène-oléfine) comme en particulier le poly(styrène-éthylène-propylène) commercialisé par la société KRATON POLYMERS sous la dénomination KRATON® G1702.

Dans le cadre de la présente invention, on préférera les copolymères triblocs SEBS ou SEPS ayant une teneur en styrène comprise entre 25 et 45 % en poids par rapport au poids dudit SEBS ou SEPS. D'une façon préférée, on utilisera des copolymères séquencés triblocs et en particulier les produits commercialisés par la société KRATON POLYMERS sous les dénominations KRATON® G1651 et KRATON® G1654.

De façon générale, l'élastomère sera utilisé dans des quantités adaptées selon la nature saturée ou insaturée de la séquence centrale oléfine du copolymère séquencé. Ainsi dans le cas d'un copolymère triblocs à séquence centrale insaturée il sera utilisé en une quantité de l'ordre de 10 à 30 % en poids, de préférence de 10 à 20 % en poids, rapportée au poids total de la composition. Dans le cas d'un copolymère triblocs à séquence centrale saturée il sera utilisé en une quantité de l'ordre de 3 à 10 % en poids, de préférence de 4 à 7 % en poids, rapporté au poids total de la composition.

Par hydrocolloïde ou particules d'hydrocolloïde, on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium, ainsi que les polymères synthétiques à base de sels de l'acide acrylique, connus sous l'appellation "superabsorbants", comme par exemple les produits commercialisés par la société BASF sous la dénomination LUQUASORB® 1003 ou par la société CIBA Specialty Chemicals sous la dénomination SALCARE® SC91 ainsi que les mélanges de ces composés.

Certains de ces superabsorbants qualifiés de « microcolloïdes » car ils présentent une taille de particules inférieure à 10 micromètres peuvent bien entendu être utilisée dans le cadre de la réalisation de la composition.

Les hydrocolloïdes préférés dans le cadre de la présente invention sont les sels de métal alcalin de la carboxyméthylcellulose, et en particulier la carboxyméthylcellulose de sodium (CMC). La taille des particules d'hydrocolloïdes est par exemple comprise entre 50 et 100 microns, notamment de l'ordre de 80 microns.

La quantité d'hydrocolloïdes incorporés dans la composition élastomère sera avantageusement de l'ordre de 2 à 20 % en poids, de préférence de 5 à 18 % en poids, de préférence encore de 8 à 18 % en poids, de préférence encore de 12 à 16 % en poids, rapportée au poids total de la composition élastomère. Les hydrocolloïdes introduits en trop grande quantité dans une couche contact perforée diminuent la capacité d'absorption d'un non tissé à base de fibres superabsorbantes au fur et à mesure que le gel se forme. En effet, la forte capacité d'absorption des hydrocolloïdes entraine un gonflement de la couche contact, si bien que les trous de la maille peuvent se boucher. Le non tissé n'absorbe plus directement les exsudats mais absorbe les exsudats présents dans la couche absorbante hydrocolloïde ce qui diminue la capacité d'absorption du matériau composite et crée des problèmes de macération.

Selon un mode préféré de réalisation, la couche contact peut comprendre un ou plusieurs élastomères choisis parmi les polymères séquencés poly(styrène-oléfine-styrène) en association avec un ou plusieurs composés plastifiants destinés à améliorer leurs propriétés d'étirement, de souplesse, d'extrudabilité ou de mise en oeuvre.

Il s'agira de préférence de composés liquides, compatibles avec la séquence centrale oléfine des copolymères séquencés utilisés.

Parmi les composés plastifiants susceptibles d'être utilisés à cet effet, on peut citer en particulier les huiles minérales plastifiantes, quelle que soit la nature de la séquence centrale. On peut également citer les polybutènes - comme par exemple les produits commercialisés par la société BP CHEMICALS sous la dénomination NAPVIS® 10 -ou encore des dérivés de phtalate tels que le dioctylphtalate ou le dioctyladipate, lorsque la séquence centrale est insaturée.

Alternativement, on peut aussi utiliser des produits de synthèse à base de mélanges liquides d'hydrocarbures saturés comme par exemple les produits commercialisés par la société TOTAL sous la dénomination GEMSEAL® et en particulier le produit GEMSEAL® 60 qui est un mélange isoparaffinique issu d'une coupe pétrolière totalement hydrogénée. On utilisera de préférence ces produits avec un copolymère triblocs comportant une séquence centrale saturée.

Dans le cadre de la présente invention, on utilisera de préférence des huiles plastifiantes et en particulier des huiles minérales formées de composés de nature paraffinique, naphténique ou aromatique ou de leurs mélanges dans des proportions variables.

Parmi les huiles plastifiantes convenant particulièrement, on peut citer :
- les produits commercialisés par la société SHELL sous les dénominations ONDINA® et RISELLA® qui sont constitués de mélanges à base de composés naphténiques et paraffiniques ;
- les produits commercialisés sous la dénomination CATENEX® qui sont constitués de mélanges à base de composés naphténiques, aromatiques et paraffiniques.

D'une façon particulièrement préférée, on utilisera une huile plastifiante minérale choisie parmi les produits commercialisés sous les dénominations ONDINA®933 et ONDINA®919.

Ces composés plastifiants peuvent être utilisés en une quantité de l'ordre de 20 à 65% en poids, de préférence de 30 à 50% en poids, rapportée au poids total de la composition élastomère hydrocolloïde.

Selon un mode de réalisation, ces compositions sont dites adhérentes : elles ont la propriété d'adhérer à la peau sans adhérer à la plaie. Elles comprennent un ou plusieurs composés dits « tackifiants » tels que ceux habituellement utilisés par l'homme de l'art dans la préparation des adhésifs sensibles à la pression à base d'élastomères. Pour une description détaillée de ces produits, on pourra se reporter à l'ouvrage de Donatas Satas "Handbook of Pressure Sensitive Technology", 3rd Edition, 1999, pages 346 à 398.

D'une façon générale, on pourra utiliser un (ou plusieurs) produit(s) tackifiant(s) qui sera (seront) incorporés(s) à la matrice élastomérique dans une proportion de l'ordre de 1 à 50 % en poids, rapportée au poids total de la composition élastomère hydrocolloïde, qui sera déterminée en fonction de la nature et de la proportion relative des autres constituants de cette dernière, pour atteindre le pouvoir micro-adhérent souhaité pour l'enveloppe.

De préférence, le(s) produit(s) tackifiant(s) représentera (représenteront) de 10 à 45 % en poids, et de préférence encore de 15 à 40 % en poids du poids total de la composition élastomère hydrocolloïde.

Les produits tackifiants susceptibles d'être utilisés dans le cadre de la présente invention pourront être choisis parmi les résines tackifiantes, les polyisobutylènes de bas poids moléculaire ou leurs mélanges.

Parmi les résines tackifiantes susceptibles d'être utilisées selon l'invention, on peut mentionner les résines polyterpènes ou terpènes modifiées, les résines de colophane, les résines hydrocarbonnées, les mélanges de résines cycliques, aromatiques et aliphatiques, ou des mélanges de ces résines.

De tels produits sont commercialisés par exemple :
- par la société ARAKAWA Chemical Industries sous la dénomination ARKON® P qui sont des résines polycyclopentadiénes hydrogénées ;
- par la société EXXON Chemical sous la dénomination ESCOREZ® et en particulier la série des résines 5000 qui sont hydrogénées.
- par la société GOODYEAR sous la dénomination WINGTACK®, et en particulier WINGTACK® 86 qui est une résine de synthèse formée de copolymères en C5/C9 ou WINGTACK® 10 qui est une résine à base de polyterpène synthétique ;
- par la société HERCULES sous la dénomination KRISTALEX® et en particulier KRISTALEX® 3085 qui est une résine à base d'alpha-méthylstyrène.

De façon générale, afin d'éviter les problèmes de coloration et de stabilité des résines insaturées, on préférera l'utilisation de résines hydrogénées, en particulier avec les copolymères triblocs à séquence centrale saturée car elles sont beaucoup plus compatibles avec ces derniers que les résines insaturées type WINGTACK que l'on utilise essentiellement avec les copolymères triblocs à séquence centrale insaturée.

Parmi ces dernières on préférera utiliser les résines ESCOREZ® de la série 5000 et tout particulièrement la résine ESCOREZ® 5380.

Les résines tackifiantes peuvent être utilisées seules ou en mélange avec d'autres produits tackifiants, de préférence dans une proportion de 10 à 50 % en poids, et plus particulièrement de 15 à 40 % en poids, rapportée au poids total de la composition.

Parmi les polyisobutylènes de bas poids moléculaire susceptibles d'être utilisés comme produits tackifiants on peut citer les polyisobutylènes ayant un poids moléculaire de l'ordre de 40 000 à 80 000 daltons, comme par exemple les produits commercialisés par la société BASF sous la dénomination OPPANOL® et en particulier les produits commercialisés sous les dénominations OPPANOL®B12 et OPPANOL®B15 ou par la societé EXXON Chemical sous la dénomination Vistanex et en particulier le grade LM-MH .

Ces polyisobutylènes pourront être utilisés seuls ou en mélange avec d'autres tackifiants en association avec les copolymères triblocs à séquence centrale insaturée. Leur proportion pourra varier dans ce cas entre 5 à 30 % en poids, et plus particulièrement de 8 à 15 % en poids, rapportée au poids total de la composition.

### Actifs

Divers composés peuvent en outre être ajoutés au matériau de la présente invention, comme, en particulier, des agents actifs ou des adjuvants couramment utilisés dans le domaine du traitement des plaies ou dans le domaine pharmacologique.

Le matériau peut contenir des principes actifs ayant un rôle favorable dans le traitement de la plaie. Ces principes actifs peuvent de préférence induire ou favoriser la cicatrisation de la plaie. D'autres agents actifs peuvent également d'être utilisés dans le cadre de l'invention, comme par exemple les agents bactéricides ou bactériostatiques, les antiseptiques, les agents anti-douleurs ou les anesthésiques locaux, les agents anti-inflammatoires, les antiprurigineux, les agents apaisants, les agents hydratants, les agents anti-oxydants, les agents dépigmentants et leurs mélanges.

De manière générale, ces actifs peuvent être choisis parmi :
- les actifs favorisant la cicatrisation tels que le Rétinol, la Vitamine A, la Vitamine E, la N-acétyl-hydroxyproline, les extraits de Centella Asiatica, la papaïne, les silicones, les huiles essentielles de thym, de niaouli, de romarin et de sauge, l'acide hyaluronique, , l'Allantoïne, - Hema'tîte (gattefossé), Vitamine C, TEGO Pep 4-17(evonik), Toniskin (silab), Collageneer (Expanscience), Timecode (Seppic), Gatuline skin repair (gattefossé), Panthenol, PhytoCellTec Alp Rose (Mibelle Biochemistry), Erasyal(libragen), Serilesine (Lipotec), Heterosides de Talapetraka (beyer), Stoechiol(codif), macarose (Sensient), Dermaveil (Ichimaru Pharcos), Phycosaccaride AI (Codif), les facteurs de croissance, la metformine, les oligosaccharides polysulfatés synthétiques ayant 1 à 4 unités oses tels qu'en particulier le sel de potassium du sucrose octasulfate (connu sous l'abréviation KSOS), commercialisé dans le produit Urgotul® Start par les Laboratoires URGO ;
- les agents bactéricides ou bactériostatiques tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, les probiotiques, sels d'argent tels que par exemple le sulfate d'argent, le chlorure d'argent, le nitrate d'argent, la sulfadiazine argentique, des ammoniums quaternaires, le polyhexaméthylène biguanide et la Chlorhexidine ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-douleurs ou les anesthésiques locaux tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les agents dépigmentants tels que l'acide kojique (Kojic Acid SL® - Quimasso (Sino Lion)), l'Arbutine (Olevatin® - Quimasso (Sino Lion)), le mélange de palmitoylpropyl de sodium et d'extrait de nénuphar blanc (Sepicalm® - Seppic), l'undécylénoyl phénylalanine (Sepiwhite® - Seppic) ;
- les antiprurigineux : hydrocotisone, enoxolone, diphenyhydramine, antihistaminique à application locale anti H1 ;
- les actifs hydratants tels que xpermoist (lipotec), Acide hyaluronique, Urée, acides gras, Glycérine, Cires, Exossine(unipex) ;
- les filtres UV tels que Parsol MCX, Parsol 1789 ;
- les agents apaisants tels que de la camomille, du bisabolol, du xanthalène, de l'acide glycyrrhébénique, tanactine (CPN), Calmiskin (Silab) ;
- les agents anti-oxydants, tels que la vitamine E.

Selon un mode préféré de réalisation, les actifs pouvant être introduits dans le matériau selon la présente invention sont de préférence choisis parmi les actifs favorisant la cicatrisation, les anti-inflammatoires et leur mélange.

Par « actif favorisant la cicatrisation », on entend tout actif capable d'intervenir favorablement à quelconque étape du processus cicatriciel et via n'importe quel type d'interaction que ce soit, c'est-à-dire par toute interaction de nature biologique, chimique ou physique avec la plaie au contact de laquelle ledit actif se trouve dispensé.

Plus particulièrement, les actifs pouvant être introduits dans le matériau selon la présente invention ou la couche contact qui peut lui être associée, sont de préférence choisis parmi les oligosaccharides polysulfatés synthétiques ayant 1 à 4 unités oses tels qu'en particulier le sel de potassium du sucrose octasulfate, l'aspirine, le sulfate d'argent, la sulfadiazine argentique, la metformine et leurs mélanges.

D'une façon générale, le matériau selon la présente invention peut comprendre des agents actifs en une quantité de 0,01 à 20 % en poids, de préférence de 1 à 15 % en poids et de préférence encore de 2 à 10 % en poids, rapportée au poids total du matériau les contenant.

Le matériau de l'invention peut également contenir des adjuvants, parmi lesquels on peut citer les matières colorantes, les charges, les absorbeurs ou piégeurs d'odeurs, les régulateurs de pH, les microcapsules ou les microsphères qui peuvent éventuellement contenir des agents actifs, la vaseline, les polymères ou les tensioactifs permettant d'optimiser la vitesse de gélification, de mouillabilité ou le relargage des actifs du matériau.

### Dispositif médical

L'invention a également pour objet un dispositif médical comprenant le matériau favorisant la cicatrisation précédemment décrit. Par « dispositif médical », on entend un équipement utilisé chez l'homme pour la prévention, le contrôle, le traitement ou l'atténuation d'une maladie ou d'une blessure.

Un tel dispositif médical peut notamment être un pansement cicatrisant, un bandage, ou un matériau composite de remplissage des plaies (« wound packing » en terminologie anglo-saxone), notamment des plaies cavitaires.

La présente invention est illustrée plus en détails dans l'exemple non limitatif suivant.

### EXEMPLE

### Préparation d'un matériau selon l'invention.

On a préparé le matériau suivant selon l'invention :
On a utilisé des fibres Verdi 1,9 dtex/40 mm commercialisées par la société Kelheim, sous forme d'un amas de fibres non transformées et directement issues du procédé de fabrication de la fibre. Ces fibres présentent une densité de charges négatives de 0,5 mmol/g et une capacité d'absorption des liquides de 12 g de sérum physiologique par gramme de fibre.

### Test du retrait de la matrice de fibrine in vitro :

Les matrices de fibrine ont été préparées selon le protocole de Brown décrit dans la publication "Fibroblast migration in fibrin gel matrices" Arm J. Pathol, 1993, 142 : 273-283.

Les composants et le mode opératoire qui ont été utilisé sont les suivants :
On a solubilisé à 37°C :
- 5 ml d'une solution aqueuse comprenant 50 millimoles d'Hépès (Catalogue Sigma-Aldrich)
- 15 mg de fibrinogène de plasma humain (catalogue Sigma-Aldrich)
- 5 millimoles de CaCl₂.

A la solution ainsi préparée, on a ajouté 50 µI de thrombine, 100 NIH de plasma humain (catalogue Sigma-Aldrich).

L'ensemble a été déposé dans une boîte de Pétri puis laissé à incuber à 37° pendant 24 heures.

Au bout de 24 heures, la matrice de fibrine est formée.

Selon un premier protocole de test, un échantillon de matériau tel que décrit précédemment est déposé sur la matrice, à température ambiante, puis retiré immédiatement.

Selon un second protocole de test, un échantillon de matériau tel que décrit précédemment est déposé sur la matrice, à température ambiante, avec un poids de 500 g pendant 30 secondes, puis est retiré.

Au retrait, on observe pour l'échantillon testé de matériau selon l'invention, selon l'un quelconque des deux protocoles suivis, que la fibrine a été décrochée de la boîte de Pétri et se trouve transférée en un seul bloc à la surface du matériau que l'on a retiré, et ce pour chaque protocole de test conduit.

En parallèle, des tests comparatifs ont été effectués.

Ainsi, un produit de type compresse non tissée, constitué de fibres de carboxymethylcellulose et commercialisé sous la dénomination Aquacel®, a été testé pour retirer une matrice de fibrine in vitro selon le second protocole décrit précédemment.

Les fibres de carboxymethylcellulose présentent une densité de charge négative de 1.5 mmol/g et une capacité d'absorption de sérum physiologique de 24,1 g par gramme de fibre.

On observe que la matrice de fibrine ne se retrouve pas décrochée de la boîte de Petri. De plus, lorsque le matériau est mis au contact d'un simulateur d'exsudats (telle qu'une solution liquide), ledit matériau constitué de fibres de carboxymethylcellulose gélifie, devenant alors très peu cohésif et ainsi rendant dans tous les cas impossible un retrait dudit matériau sans déchirure.

Un autre produit de type compresse non tissée et constituée cette fois de fibres d'alginate, commercialisé sous la dénomination Algostéril®, a été testé pour retirer une matrice de fibrine in vitro selon le second protocole décrit précédemment.

Les fibres d'alginate présentent une densité de charge négative de 5.10 mmol/g et une capacité d'absorption de sérum physiologique de 12.8 g par gramme de fibre.

On observe ici que la matrice de fibrine ne se retrouve pas décrochée de la boîte de Pétri bien que le retrait dudit produit se fasse en un seul morceau. L'accroche de la fibrine est nulle.

## Revendications

1. Matériau comprenant au moins une fibre électronégative présentant une densité de charges négatives comprise entre 0,01 et 5 mmol/g et une capacité d'absorption des liquides comprise entre 10 et 20 g de sérum physiologique par gramme de fibre pour son utilisation dans la cicatrisation des plaies.

2. Matériau selon la revendication 1 pour son utilisation dans la détersion des plaies.

3. Matériau selon l'une quelconque des revendications précédentes, **caractérisé en ce que** la fibre électronégative présente une densité de charges négatives comprise entre 0,05 et 4 mmol/g, de préférence entre 0,1 et 2 mmol/g.

4. Matériau selon l'une quelconque des revendications précédentes, **caractérisé en ce que** la fibre électronégative est une fibre polymérique, le polymère constituant la fibre étant choisi parmi les polyesters, les polyamides, les polyoléfines et leurs copolymères, le polyuréthane, les polymères acryliques, les polyacrylates et leurs copolymères, les polymères cellulosiques , et des mélanges de ceux-ci.

5. Matériau selon la revendication précédente, **caractérisé en ce que** le polymère constituant la fibre électronégative est un polymère cellulosique de préférence un dérivé cellulosique ou de la viscose, ou un polymère acrylique, de préférence un copolymère d'acrylonitrile et de chlorure de vinyle.

6. Matériau selon l'une quelconque des revendications précédentes, **caractérisé en ce que** la fibre électronégative est obtenue soit par incorporation de particules ayant des propriétés échangeuses de cations ou de polyélectrolytes lors de la fabrication de ladite fibre, soit par synthèse chimique d'un polymère présentant des groupements ioniques appropriés.

7. Matériau selon l'une quelconque des revendications précédentes, **caractérisé en ce que** la fibre électronégative est obtenue par incorporation de résines échangeuses d'ions porteuses de groupements sulfonate et/ou par incorporation de polyélectrolytes porteurs de groupements carboxylate dans une matrice polymérique de viscose ou par réaction d'un polymère modacrylique, et en particulier d'un copolymère acrylonitryle/chlorure de vinyle avec une amine présentant un groupement échangeur d'ions.

8. Matériau selon l'une quelconque des revendications précédentes, **caractérisé en ce qu'**il se présente sous la forme d'un non tissé, d'un tissé, ou d'un tricot.

9. Matériau selon l'une quelconque des revendications précédentes, **caractérisé en ce qu'**il présente un grammage supérieur à 75 g/m².

10. Matériau selon l'une quelconque des revendications précédentes, **caractérisé en ce qu'**il est partiellement recouvert d'une couche contact sur la face du matériau destinée à venir en contact avec la plaie, ladite couche comprenant des ouvertures permettant le passage des exsudats de la plaie.

11. Matériau selon l'une quelconque des revendications précédentes, **caractérisé en ce qu'**il contient des principes actifs favorisant la cicatrisation des plaies.

12. Dispositif médical comprenant un matériau selon l'une quelconque des revendications 1 à 11, ledit dispositif étant un pansement cicatrisant ou un bandage.

13. Dispositif médical comprenant un matériau selon l'une quelconque des revendications 1 à 11, ledit dispositif étant un matériau composite de remplissage des plaies cavitaires.

## Patentansprüche

1. Material, welches wenigstens eine elektronegative Faser umfasst, die eine Dichte negativer Ladungen zwischen 0,01 und 5 mmol/g und eine Absorptionsfähigkeit für Flüssigkeiten zwischen 10 und 20 g physiologisches Serum pro Gramm Faser zur Verwendung in der Wundheilung aufweist.

2. Material nach Anspruch 1 zur Verwendung in der Wundreinigung.

3. Material nach einem der vorhergehenden Ansprüche, **dadurch gekennzeichnet, dass** die elektronegative Faser eine Dichte negativer Ladungen zwischen 0,05 und 4 mmol/g, bevorzugt zwischen 0,1 und 2 mmol/g aufweist.

4. Material nach einem der vorhergehenden Ansprüche, **dadurch gekennzeichnet, dass** die elektronegative Faser eine Polymerfaser ist, wobei das die Faser bildende Polymer ausgewählt ist aus Polyestern, Polyamiden, Polyolefinen und deren Copolymeren, Polyurethan, Acrylpolymeren, Polyacrylaten und deren Copolymeren, Cellulosepolymeren, und Mischungen daraus.

5. Material nach dem vorhergehenden Anspruch, **dadurch gekennzeichnet, dass** das die elektronegative Faser bildende Polymer ein Cellulosepolymer ist, vorzugsweise ein Cellulosederivat oder Viskose, oder ein Acrylpolymer, vorzugsweise ein Copolymer aus Acrylnitril und Vinylchlorid.

6. Material nach einem der vorhergehenden Ansprüche, **dadurch gekennzeichnet, dass** die elektronegative Faser entweder durch Einbetten von Partikeln mit Kationen- oder Polyelektrolytaustauschmerkmalen bei der Herstellung der Faser erhalten wird oder durch chemische Synthese eines Polymers, das geeignete ionische Einheiten umfasst.

7. Material nach einem der vorhergehenden Ansprüche, **dadurch gekennzeichnet, dass** die elektronegative Faser durch Einbetten von Sulfonatgruppen tragenden Ionenaustauschharzen und/oder durch Einbetten von Carboxylatgruppen tragenden Polyelektrolyten in eine polymere Viskosematrix erhalten wird oder durch die Reaktion eines Modacrylpolymers, und insbesondere eines Acrylnitril-/Vinylchlorid-Copolymers, mit einem Amin, welches eine Ionenaustauschgruppe aufweist.

8. Material nach einem der vorhergehenden Ansprüche, **dadurch gekennzeichnet, dass** es in Form eines Vlieses, Gewebes oder Gewirks vorliegt.

9. Material nach einem der vorhergehenden Ansprüche, **dadurch gekennzeichnet, dass** es ein Grammgewicht größer 75 g/m² aufweist.

10. Material nach einem der vorhergehenden Ansprüche, **dadurch gekennzeichnet, dass** es teilweise mit einer Kontaktschicht auf derjenigen Seite des Materials bedeckt ist, die mit der Wunde in Kontakt kommen soll, wobei die Schicht Öffnungen umfasst, die den Durchfluss von Wundexsudaten erlauben.

11. Material nach einem der vorhergehenden Ansprüche, **dadurch gekennzeichnet, dass** es Wirkstoffe umfasst, die die Wundheilung begünstigen.

12. Medizinprodukt, welches ein Material nach einem der Ansprüche 1 bis 11 umfasst, wobei das Produkt ein Wundverband oder eine Bandage ist.

13. Medizinprodukt, welches ein Material nach einem der Ansprüche 1 bis 11 umfasst, wobei das Produkt ein Verbundmaterial zum Füllen von Wundhöhlen ist.

## Claims

1. Material comprising at least one electronegative fibre having a negative charge density between 0.01 and 5 mmol/g and a liquid absorption capacity between 10 and 20 g of physiological saline solution per gram of fibre for use in the healing of wounds.

2. Material according to claim 1 for use in the cleansing of wounds.

3. Material according to any one of the preceding claims, **characterised in that** the electronegative fibre has a negative charge density between 0.05 and 4 mmol/g, preferably between 0.1 and 2 mmol/g.

4. Material according to any one of the preceding claims, **characterised in that** the electronegative fibre is a polymeric fibre, the polymer forming the fibre being chosen from polyesters, polyamides, polyolefins and the copolymers thereof, polyurethane, acrylic polymers, polyacrylates and the copolymers thereof, cellulosic polymers, and mixtures thereof.

5. Material according to the preceding claim, **characterised in that** the polymer forming the electronegative fibre is a cellulosic polymer preferably a derivative of cellulose or viscose, or an acrylic polymer, preferably a copolymer of acrylonitrile and vinyl chloride.

6. Material according to any one of the preceding claims, **characterised in that** the electronegative fibre is obtained either by incorporating particles having cation exchanging properties or polyelectrolytes during the manufacture of said fibre, or by chemical synthesis of a polymer having suitable ionic groups.

7. Material according to any one of the preceding claims, **characterised in that** the electronegative fibre is obtained by incorporating ion exchange resins bearing sulphonate groups and/or by incorporating polyelectrolytes bearing carboxylate groups in a polymeric viscose matrix or by reacting a modacrylic polymer, and in particular an acrylonitrile/vinyl chloride copolymer with an amine having an ion exchanging group.

8. Material according to any one of the preceding claims, **characterised in that** it is presented in the form of a nonwoven, woven, or knitted fabric.

9. Material according to any one of the preceding claims, **characterised in that** it has a grammage greater than 75 g/m².

10. Material according to any one of the preceding claims, **characterised in that** it is partially covered with a contact layer on the side of the material intended to come into contact with the wound, said layer comprising openings allowing the passage of exudates from the wound.

11. Material according to any one of the preceding claims, **characterised in that** it contains active substances promoting wound healing.

12. Medical device comprising a material according to any one of claims 1 to 11, said device being a wound-healing dressing or a bandage.

13. Medical device comprising a material according to any one of claims 1 to 11, said device being a composite cavity wound packing material.
